# EUROPEAN PATENT SPECIFICATION

(11) **EP 3 099 331 B1**
(45) Date of publication and mention of the grant of the patent: **10.03.2021**
(21) Application number: 15702553.7
(22) Date of filing: 28.01.2015
(51) Int. Cl.: A61K 39/00, A61K 38/17, A61K 47/10, A61K 47/12, A61K 47/14, A61K 47/32, A61K 47/64, A61K 9/00, A61P 17/00, A61P 27/02, A61P 43/00

(54) **TRANSMEMBRANE DRUG DELIVERY SYSTEM**
SYSTEM ZUR TRANSMEMBRANEN ARZNEIMITTELABGABE
SYSTEME D'ADMINISTRATION DE MEDICAMENT TRANSMEMBRANE

(30) Priority: 28.01.2014 GB 201401453
(43) Date of publication of application: 07.12.2016
(60) Divisional application: 20216082.6
(73) Proprietor: MacRegen, Inc., Birmingham, Alabama 35233 (US)
(72) Inventor: STAMBOULIS, Artemis, Birmingham West Midlands B15 2TT (GB); LOGAN, Ann, Birmingham West Midlands B15 2TT (GB); DE COGAN, Felicity Jane, Birmingham West Midlands B15 2TT (GB); SCOTT, Robert, Edgbaston Birmingham West Midlands B15 2TT (GB); PEACOCK, Anna Frances Acushla, Birmingham West Midlands B15 2TT (GB); HILL, Lisa Jayne, Birmingham West Midlands B15 2TT (GB)
(74) Representative: Lahrtz, Fritz
(86) International application number: PCT/GB2015/050190
(87) International publication number: WO 2015/114324

(56) References cited:
- WO-A2-97/23240
- WO-A2-98/52614
- US-A1- 2003 022 831
- KAMEI N ET AL: "Permeation characteristics of oligoarginine through intestinal epithelium and its usefulness for intestinal peptide drug delivery", JOURNAL OF CONTROLLED RELEASE, ELSEVIER, AMSTERDAM, NL, vol. 131, no. 2, 21 October 2008 (2008-10-21), pages 94-99, XP025478794, ISSN: 0168-3659, DOI: 10.1016/J.JCONREL.2008.07.016 [retrieved on 2008-07-18]
- MITCHELL D J ET AL: "POLYARGININE ENTERS CELLS MORE EFFICIENTLY THAN OTHER POLYCATIONIC HOMOPOLYMERS", JOURNAL OF PEPTIDE RESEARCH, BLACKWELL PUBLISHING LTD, OXFORD; GB, vol. 56, no. 5, 1 November 2000 (2000-11-01), pages 318-325, XP001094094, ISSN: 1397-002X, DOI: 10.1034/J.1399-3011.2000.00723.X
- UEMURA SHIRO ET AL: "SHORT POLYMERS OF ARGININE RAPIDLY TRANSLOCATE INTO VASCULAR CELLS - EFFECTS ON NITRIC OXIDE SYNTHESIS", CIRCULATION JOURNAL, JAPANESE CIRCULATION SOCIETY, KYOTO, JP, vol. 66, no. 12, 1 January 2002 (2002-01-01), pages 1155-1160, XP009077981, ISSN: 1346-9843, DOI: 10.1253/CIRCJ.66.1155 cited in the application
- Ikuhiko Nakase ET AL: "Efficient Intracellular Delivery of Nucleic Acid Pharmaceuticals Using Cell-Penetrating Peptides", ACCOUNTS OF CHEMICAL RESEARCH., vol. 45, no. 7, 17 July 2012 (2012-07-17), pages 1132-1139, XP055658349, US ISSN: 0001-4842, DOI: 10.1021/ar200256e

## Description

The invention provides a transmembrane, transmucosal or transepithelial delivery system for transporting pharmaceutically active moieties across, for example, skin, the surface of the eye, or mucosal membranes. The delivery systems are for use in a method for treating an eye disease.

There is a wide range of applications for delivering pharmaceutically active moieties across mucosal and epithelial surfaces. These include the treatment for acute conditions, such as burns, trauma and infection; and chronic treatments for conditions that include debilitating eye diseases such as glaucoma, age-related macular degeneration and retinal scarring after ocular surgery. To show proof of principle, we have focused on delivery of an anti-scarring drug into the eye to target one of the pathological processes occurring in Glaucoma.

Uemura et al (Circulation J. (2202), 66, 1155-1160) describe the translocation of short polymers of arginine into cultured vascular smooth muscle cells. Heptamers of arginine were especially rapidly transported into cells where they were used to study the effects of the polymers on nitric oxide synthesis. The polymers were observed to rapidly translocate through cytoplasmic and nuclear membranes efficiently.

Other so called cell penetrating peptides have also been used to transport, for example, DNA or siRNA into cultured cells, but not in a more complex system of several layers of cells as found in transmembrane systems.

The inventors have unexpectedly found that it is possible to transport molecules, such as the Decorin, across the dermis, cornea or skin of patients by using them in combination with a polypeptide having basic amino acids.

Glaucoma is a chronic neurodegenerative disease of the retina and optic nerve and is the second leading cause of blindness worldwide following cataracts (Resnikoff et al., 2004). However, unlike cataracts, left untreated glaucoma results in irreversible blindness (Weinreb and Khaw, 2004). Globally 66 million people have glaucoma and 6.8 million people are permanently blind in both eyes from this disease (Weinreb and Khaw, 2004). Glaucoma's are a group of optic neuropathies which are characterised by progressive death of retinal ganglion cells (RGC) and their axons (which form the optic nerve) leading to visual defects and loss The main, or most common, type of glaucoma is Primary Open Angle Glaucoma (POAG). Ocular hypertension, or increased intraocular pressure (IOP), is the main risk factor for the onset and exacerbation of POAG (Junglas et al., 2012) and, although the pathological process is not fully understood, it is thought that ocular hypertension leads to a continuous compression of axons at the optic nerve head leading to RGC death by apoptosis. Other risk factors for the development of POAG include increasing age, ethnicity and family history of glaucoma (Morrison & Pollack, 2002).

Pharmacological and surgical treatments for POAG focus solely on lowering IOP (to relieve compression on the RGC axons) as currently this is the only controllable risk factor, but does not address the underlying pathological process of glaucoma or provide any direct neuroprotection for RGC death (Marquis et al. 2005). Although some IOP lowering agents provide good symptomatic control, patient's vision deteriorates with time as RGC are lost, leading to the progressive blindness typical of the condition.

Although the aetiology of POAG is not understood, it is known that resistance to aqueous humour outflow through the TM increases IOP, which slowly leads to RGC death and degeneration of neurons in the lateral geniculate nucleus and the visual cortex. RGC degeneration occurs slowly from increased pressure on the lamina cribosa and RGC axons leading to altered cell morphology and death, by apoptosis, due to mechanical stresses blocking proteins and trophic factor transport along axons (Fraser, 2005; Weinreb and Khaw, 2004). The loss of RGC is correlated with loss of vision and it is surmised that prevention of RGC death by neuroprotection either directly using anti-apoptotic agents or indirectly by reducing outflow resistance will provide better treatments for patients.

The exact mechanisms which lead to resistance of outflow from the TM are not known. It is thought that structural changes within the TM leads to increased levels of extracellular matrix (ECM) deposition (Junglas et al., 2012). Scarring, or fibrosis, is a wound healing response following cellular or metabolic insults. Increased levels of TM fibrosis have been observed in patients and this is suggested to be responsible for the ocular hypertension seen in POAG (Borras, 2003; WuDunn, 2009). Increased ECM deposition within the eye occurs with age in the normal population but this process is thought to be more aggressive, and occur sooner, in patients with glaucoma (Tektas and Lütjen-Drecoll, 2009).

Aberrant growth factor signalling is thought to be responsible for excess ECM deposition and reduced ECM degradation within the TM and SC. Transforming growth factor (TGF)-β's are multifunctional cytokines that are up-regulated in response to inflammation and injury (Logan et al., 1992). TGF-β 1&2 are the main cytokines involved in scarring within the CNS (Logan et al., 1992) and are thought to play a role in glaucoma by increasing TM fibrosis (Shepard et al. 2010; Junglas et al, 2012). Higher levels of TGF-β were detected in aqueous humour samples of patients with POAG compared to non-glaucomatous controls (Gottanka, 2004). Other studies have shown that TGF-β antagonists (e.g. Decorin) prevent and reduce scar formation within the CNS (Logan et al., 1999). Hence antagonising the TGF-β sub10 family of cytokines may be a promising avenue for a better treatment for glaucoma as the strategy targets the pathology rather than just the symptoms.

Decorin is a naturally occurring ECM dermatan sulphate glycoprotein of approx 70kD (Brandan et al., 1992). It interacts with ECM proteins, cytokines and cell surface receptors including TGP-β (Logan et al. 1999), epidermal growth factor receptor (Biglari et al., 2004) and vascular endothelial growth factor (Grant et al., 2002). It is found in tissue rich in fibrillar collagen where it plays an important role in its formation. Decorin sequesters TGF-βs and prevents them binding to their receptors and has been shown to reduce scar formation and inflammation following cerebral injury (Logan et al., 1999) and spinal cord damage (Davies et al, 2004).

A conjugate comprising a delivery enhancing transporter comprising polyarginine of 5 to 25 arginine residues and a compound for delivery of drugs or other agents across epithelial tissues including ocular tissues is provided in US 2003/0022831 A1.

The invention provides a transmembrane, transmucosal or transepithelial delivery system for use in a method of treating an eye disease, wherein the system comprises:
a pharmaceutically active agent which is a negatively charged polypeptide or glycoprotein; and
a polyarginine of 4 to 20 amino acids in length;
wherein the pharmaceutically active agent is mixed with or non-covalently bound to the polyarginine.
In an embodiment, the polyarginine consists of 6, 7 or 10 arginine residues.

Disclosed is a transmembrane/transepithelial delivery system comprising a pharmaceutically effective moiety; and a polypeptide of up to 20 amino acids in length, the polypeptide comprising a continuous region of at least 2, more typically at least 4 basic amino acids. Typically the polypeptide comprises at least 4, at least 5, at least 6, at least 7, at least 8, at least 9, at least ten, or less than 19, less than 18, less than 17, less than 16, less than 15, less than 14, less than 13, less than 12, less than 11 or less than 10 amino acids in length.

According to the invention, the polypeptide is a polyarginine of 4 to 20 amino acids in length, in an embodiment thereof of 6, 7 or 10 arginine residues.

Amino acids may be D or L amino acids, preferably L amino acids, and may be naturally or non naturally occurring. That is, they may occur naturally within the cell or may be artificial or synthetic peptides.

Typically the amino acids making the polypeptide are joined by means of peptidic bonds to each other.

The polypeptides may be covalently or non-covalently bound to a support, such as titanium or hydroxyapatite, or a polymer or hydrogel, for example by grafting onto the polymer or hydrogel or, for example, via a binding moiety comprising an amino acid sequence.

Typically the polypeptide has formula:

(B)ₙ (A)ₘ

where B is a basic amino acid
A may or may not be present and where present is an acidic amino acid, and
m and n and integers and
n is at least 4.

Typically m is less than n.

Typically the total number of amino acids is 6, 7, 8, 9 or 10.

n may be 6 and m may be 4, for example.

Typically all of the amino acids are basic amino acids. 1, 2 or 3 non basic or non acidic amino acids may also be present.

Basic amino acid may be selected from arginine, lysine and histidine, or mixtures thereof. Typically the basic amino acid is arginine. Typically the polypeptide is a polyarginine consisting of 6, 7 or 10 arginine residues.

The acidic amino acid may be selected from aspartate and glutamate. Typically the acidic amino acid is glutamate. The acid amino acid residues may allow the polypeptide to bind to a support such as hydroxyapatite. The acidic amino acids may be replaced by an alternative amino acid sequence to allow it to bind to metals such as titanium or electrostatically to the pharmaceutically active moiety. The sequence may be, for example, RKLPDA to bind to titanium.

The polypeptide may be grafted or incorporated onto a polymer or hydrogel or similar material.

According to the invention, the pharmaceutically active moiety is a negatively charged polypeptide or glycoprotein. It may, for example, be an anti-inflammatory compound or an antimicrobial compound.

The pharmaceutically active moiety may have a molecular weight of up to, for example, 500 kDa, 30-400 kDa, 100-200 kDa or 150 kDa

The polypeptide may form nanosomes of between 2 -5,000 nm, more typically 10 - 1,000 nm or 100 - 500 nm diameter This is typically when in combination with the pharmaceutically active moiety, so the total size of the polypeptide - pharmaceutically active complex is typically between 2-5,000nm, 10-1,000nm or 100-500nm diameter. Alternatively, the polypeptides themselves form nanosomes which bind to larger pharmaceutically active moieties.

According to the invention, the pharmaceutically active agent is mixed with or non-covalently bound to the polyarginine. The pharmaceutically active moiety and polypeptide may, for example, be ionically bound together. Disclosed is that the pharmaceutically active moiety and polypeptide may be covalently bound. They may be covalently bound, for example, through an attachment moiety which covalently attaches the polypeptide and pharmaceutically active moiety. The attachment moiety may comprise: succinimidyl succinate, N-hydroxy succinimide, succinimidyl propionate, succinimidyl butanoate, propionaldehyde, acetaldehyde, tresylate, triazine, vinyl sulfone, benzotriazole carbonate, maleimide, pyridyl sulfide, iodoacetamide, succimidyl carbonate or avidin/biotin.

Such moieties are generally known in the art for the attachment of compounds to proteins or other pharmaceutically active moieties.

For example, the polypeptide may comprise an attachment moiety, optionally attached to the polypeptide via a linker, such as an alkyl moiety. The polypeptide may be then reacted with the pharmaceutically active moiety to covalently link the polypeptide to the pharmaceutically active moiety.

The system is suitable for transporting pharmaceutically active agents across single and multi-layered membranes, for example, the cornea of the surface of the eye, and indeed mucous membranes or indeed skin Depending on the area to be treated, one or more additional compounds may be provided to assist with the penetration of the material or to make the material more suitable for the intended use.

For example, the system may be provided as in the form of eye drops, for example in a saline solution for applying to the surface of the eye to allow the pharmaceutically active moiety to be transported across the membrane of the eye into the eye itself.

Disclosed is the system in the form of drops, for example for applying to the mucous membrane of the nose, or indeed as an ointment for applying to the surface of the nose. The system may be provided in the form of a suppository to allow the pharmaceutically active agent to be absorbed through the mucous membranes of the rectum, vagina, or urethra. Typically such suppositories comprise a greasy base, such as cocoa butter, in which the active ingredient and other excipients are dissolved. The grease will melt at body temperature to release the active ingredients. Other suppositories may be made from a water soluble base such as polyethylene glycol, glycerin or gelatin.

Disclosed is the system in the form of a patch which is applied to the surface of the skin.

Such patches are generally known.

Transdermal patches are medicated adhesive patches that are placed on the skin to deliver a specific dose of medication through the skin and into the blood stream. The pharmaceutically active moiety and polypeptide are disclosed to be present within, for example, an adhesive layer. The adhesive layer not only serves to adhere various layers together, along with the entire system to the skin, but is also responsible for the releasing of the pharmaceutically active moiety and polypeptide onto the skin. Multi-layer drug systems are also known which use a separate layer for control of release of drug from a reservoir onto the skin.

The system may be used in combination with one or more additional penetration enhancers

The penetration enhancer may be selected from but not exclusively, for example, polyethylene glycol, fatty acid esters, diacids and monomethyl esters.

The invention also provides the systems of the invention for use in the treatment of eye diseases. The eye diseases include, for example, the treatment of glaucoma, proliferative vitreoretinopathy and age related macular degeneration scarring. It may also be used to treat burns and other wounds on both the eye and skin.

The treatment comprises applying a pharmaceutically effective amount of the system of the invention.

Disclosed is a method of transporting a pharmaceutically effective moiety across skin, the surface of an eye or a mucous membrane, comprising applying to the skin or membrane the system.

The invention will now be described by way of example only with reference to the following figures:
**Figure 1** shows an initial ELISA data for the passage of Decorin across the cornea when aided by the nanosome.
**Figure 2** shows OCT images of nanosome/Decorin crossing the eye.
**Figure 3** shows TEM image of nanosome drops.
**Figure 4** shows Decorin concentration after treatment of nanosome drops to the cornea of rats.

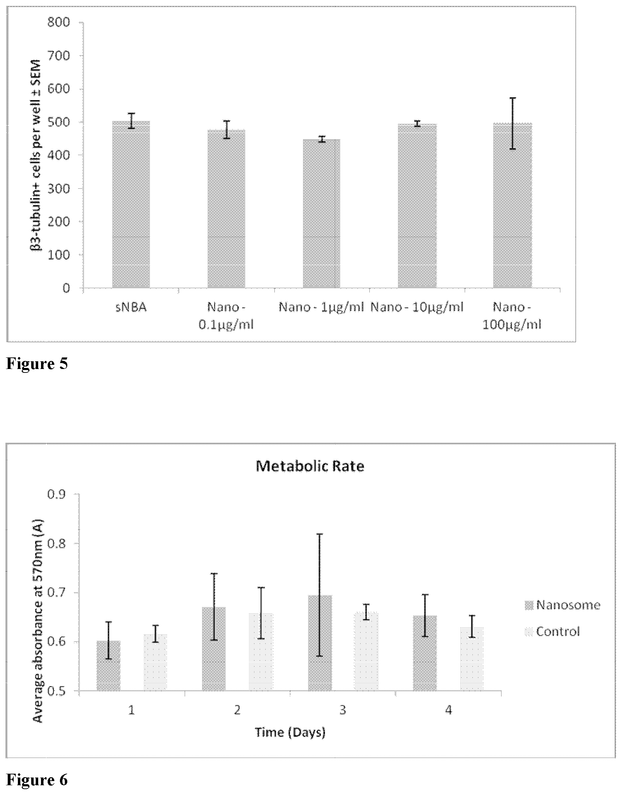

**Figure 5** shows cytotoxic effects of nanosomes against Retinal Ganglion Cells.
**Figure 6** shows metabolic activity of MF cells when treated with nanosome.
**Figure 7** shows DNA assay on cell samples to give viability.
**Figure 8** shows CD data for nanosome peptide.
**Figure 9** shows the concentration of Avastin® in eye after treatment with saline (PBS), nanosomes, Avastin® and nanosomes combined with Avastin® on application to enucleated pig eyes.

### Methods

Peptides were synthesised using the solid phase peptide synthesis method (SPPS). This used resin beads as a solid phase support and Fmoc (Fluorenylmethyloxycarbonyl) protection chemistry which ensured that the correct reactions occurred. Wang beads were preattched with an initial amino acid on the bead. All the amino acids were protected on the amine group with an Fmoc protection group. The Fmoc group was cleaved using piperidine which gave the free amine group on the amino acid. Once the Fmoc group was removed a second Fmoc protected amino acid was added to the resin. This coupling reaction uses HBTU (*O-*(Benzotriazol-1-yl)-*N,N,N',N'*-tetramethyluronium hexafluorophosphate) as a coupling reagent and was facilitated by DIPEA (diisopropylethylamine) which activated the carboxyl group and increased the reaction speed. This reaction gave rise to amide bond formation between the two amino acids and a dipeptide with a protected amine terminus was formed. This was then deprotected and reacted further until the full amino acid sequence was achieved. This peptide synthesis method has been utilised to create several different peptides, 1) RRRRRRRRRR, 2) RRRRRR, 3) RRRRRR-Linker-FITC, 4)RRRRRR-EEEE, 5)RRRRRREEEE-Linker-FITC. The peptides were analysed using mass spectrometry which demonstrated the presence of the peptides in each group and by HPLC to ascertain the purity of the peptides. Covalent Decorin/nanosome drops (not according to the invention) were synthesised using a maleimide linker to crosslink the amine group of the nanosome with a thiol group on the Decorin protein sequence.

Spectroscopy was used to try and determine the binding interactions between Decorin and the RRRRRR-Linker-FITC. The spectra of Decorin was monitored using UV-Vis spectroscopy. The nanosomes were titrated into the Decorin solution with continuous UV monitoring. Peak shifts were observed on the addition of 2x10⁻⁷ M

**Table 1 UV shift of nanosome-Decorin binding**

| Decorin | Decorin + nanosome |
|---|---|
| | |
| 208.5 | 203.5 |
| 205.5 | 200.5 |

This shift demonstrated that the nanosomes were binding with the Decorin. The shift pattern suggested that the peptide: Decorin was binding in a 2:1 ratio.

The RRRRRR-Linker FITC peptide was then taken forward for *in vitro* testing.

### Initial in-vivo testing

5mg of nanosome (NS) was diluted in 500ul PBS and 5mg of NS diluted in 500ul 5mg/ml Decorin.

Four male Sprague Dawley rats were given 3 drops to each eye. All left eyes were given PBS drops and all right eyes given Decorin drops. Two minutes after rats were killed using increasing concentrations of CO2 over 8 minutes. 10ul of Aqueous humour (AqH) was removed from the anterior segment of each eye using glass micropipettes and placed into Eppendorf tubes ready for analysis. All AqH from eyes treated with PBS were pooled together to a final volume of 40ul. All AqH from eyes treated with Decorin were pooled together to a final volume of 40ul.

The AqH samples were analysed to ascertain Decorin concentrations using the commercially available human Decorin ELISA kit (R&D Systems, Cat: DY143). The ELISA protocol was as per kit instructions. No Decorin was detected in the AqH sample following external NS+PBS drops. Following the external NS+Decorin drop, 6.47ng/40ul of Decorin was detected in the AqH.

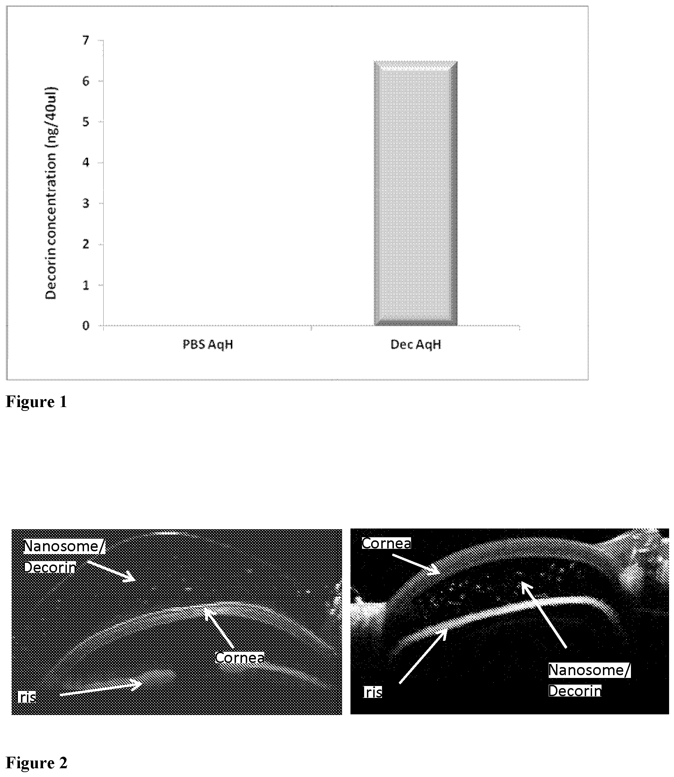

As Figure 1 shows, when nanosome + Decorin drops are applied to the front of the cornea. Decorin is rapidly found in the aqueous humour The level of Decorin found in the aqueous humour is significantly higher than the control. This demonstrates that the Decorin is being carried across the cornea and into the eye. This experiment utilised the electrostatic drop with no covalent linkage between the nanosome and Decorin.

As Figure 2 demonstrates when the nanosome /Decorin mixture is applied to the eye it diffuses across the cornea and into the aqueous humour. In the first image the nanosome/Decorin drop can be seen as a liquid droplet on the front of the cornea at time 0. After 6 minutes the fluorescently tagged nanosome/Decorin can be seen in the anterior chamber. As the nanosome/Decorin can be seen as small dots visible to the eye it was hypothesised that the nanosome/Decorin was aggregating together in structures big enough to be visible on the OCT. Dynamic light scattering (DLS) was carried out on the solution to find out if structures were forming. The DLS demonstrated a range of sizes of particles from 2nm to 5000nm. This showed that the nanosome + Decorin were forming aggregates with each other but there did not appear to be any specific size to the aggregates and this did not affect the bioactivity or clearance.

The aggregates were imaged using Transmission Electron Microscopy (TEM) This showed a number of aggregates of different sizes on the micro and the nanoscale. There was no specific order to the structures and a broad range of sizes were seen. These structures probably occur as the nanosome peptides bind more than one molecule of Decorin at a time bringing them into close proximity and causing aggregation.

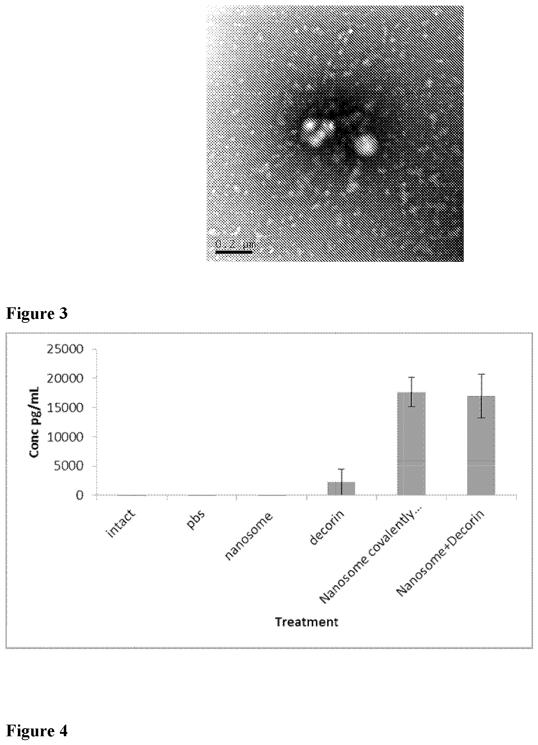

A second ELISA test was then carried out to determine levels of Decorin carried across the cornea. This was to compare the electrostatic and the covalent drop and to cover all controls (Figure 4).

Figure 4 shows that using the electrostatic drop where the nanosome and the Decorin are not covalently cross linked does significantly increase the level of Decorin which passes across the cornea from the topical application.

The cells were studied against several cell lines for any cytotoxic effects they might induce. Dissociated retinal cultures were prepared from rats and plated onto culture plates. The cells were treated with nanosome peptide and then incubated for 72 hours and fixed with formaldehyde. The cells were stained with β3 tubulin and imaged on the fluorescence microscope. The positive cells were counted and comparisons were taken. The data shows that there is no significant difference between wells treated with the nanosome peptide and the control and that increasing the nanosome peptide concentration upto 100 µg/mL did not have any effect on cell viability.

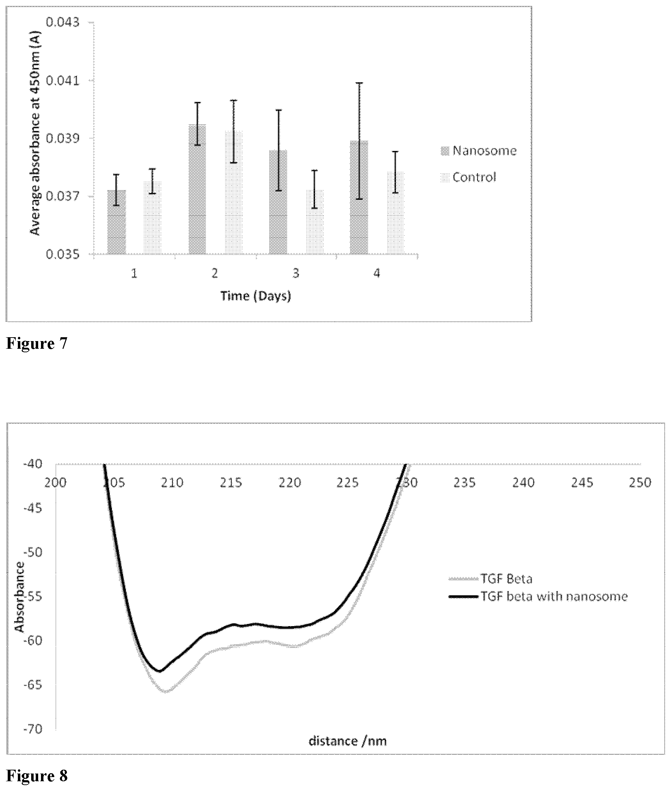

The nanosome toxicity was also tested against primary rat meningeal fibroblast cells. The cells were isolated from Sprague-Dawley Rats The leptomeninges was removed from the rat and the meningeal fibroblast cells were isolated from the tissue. The cells were then plated out and treated with nanosome. The cell media was supplemented with resazurin for 2 hours and then harvested and the media fluorescence read at 570 nm. The cell samples were then frozen in deionised water for DNA analysis (Figure 7).

Figure 7 shows that the nanosome peptide did not have a significant effect on the number of cells in the sample, with no significant differences observed between the peptide and the control. This supports the work carried out in the retinal cultures that the nanosomes are not toxic.

Decorin interacts with TGF β and other bioactive molecules in tissues to modulate their activity and to provide a clinical result In order to understand if there are any interactions between nanosomes and TGF beta, circular dichroism was carried out to monitor structural shifts in TGF beta in the presence of the peptide. A slight shift is seen when the peptide is titrated into the TGF beta solution. This indicates that the nanosome could induce a structural change in the TGF beta. As TGF beta and nanosomes are both positively charged this cannot be due to electrostatics and suggests that the nanosomes are targeting a specific site on the TGF beta molecule.

Further studies utilising the nanosome also show that it is a highly effective broad spectrum antibacterial agent. It is effective at killing both gram positive and gram negative bacteria and was particularly selective against pseudomonas aeruginosa with a MIC of 0.03 mg/mL.

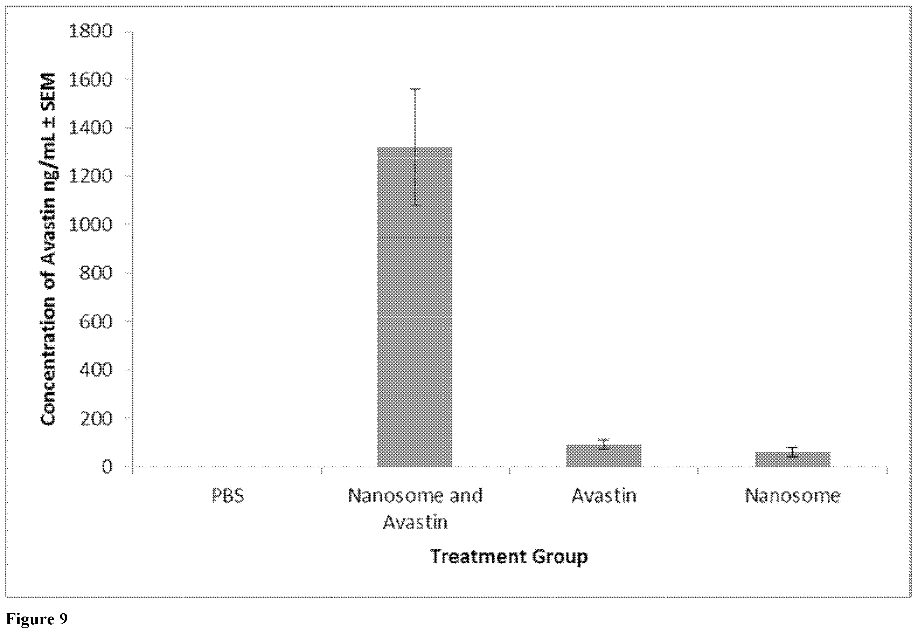

Further studies looked at the transport of Avastin® across pig eyes. Freshly enucleated pig eyes had applied nanosomes, Avastin®, nanosomes and Avastin® or saline to the ocular surface. These were left for two hours and the inventors then removed the vitreous and retina. They then used an ELISA to measure the concentration of the Avastin® in the vitreous. Concentrations are shown in Figure 9.
Resnikoff S, Pascolini D, Etya'ale D, Kocur I, Pararajasegaram R, Pokharel GP, Mariotti SP. Global data on visual impairment in the year 2002. B World Health Organ. 2004;82:844-851
Weinreb R N, T KP. Primary open-angle glaucoma. Lancet. 2004;363:1711-1720
Junglas B, Kuespert S, Seleem AA, Struller T, Ullmann S, Bösl M, Bosserhoff A, Köstler J, Wagner R, Tamm ER, Fuchshofer R. Connective tissue growth factor causes glaucoma by modifying the actin cytoskeleton of the trabecular meshwork. The American Journal of Pathology. 2012; 180:2386-2403
Morrison JC, Pollack IP. Glaucoma: Science and practice. New York: Thieme; 2003.
Marquis RE, Whitson JT. Management of glaucoma: Focus on pharmacological therapy. Drugs & amp; Aging. 2005,22:1-21 Fraser 2005
Borrás T. Gene expression in the trabecular meshwork and the influence of intraocular pressure. Progress in Retinal and Eye Research. 2003,22:435-463
WuDunn D. Mechanobiology of trabecular meshwork cells. Experimental Eye Research. 2009;88:718-723
Tektas OY and Lutjen-Drecoll E.Structural changes of the Trabecular Meshwork in different kinds of glaucoma. Experimental Eye Research. 2009; 88:769-775
Logan A, Frautschy SA, Enhanced expression of transforming growth factor [beta] 1 in the rat brain after a localized cerebral injury. Brain Research. 1992; 587:216-225
Gottanka J, Chan D, Eichhorn M, Lütjen-Drecoll E, Ethier CR. Effects of tgf-β2 in perfused human eyes. Invest Ophth Vis Sci. 2004;45:153-158
Logan A, Baird A, Berry M. Decorin attenuates gliotic scar formation in the rat cerebral hemisphere. Experimental Neurology 1999;159:504-510
Brandan E, Fuentes ME, Andrade W. Decorin, a chondroitin/dermatan sulfate proteoglycan is under neural control in rat skeletal muscle. Journal of Neuroscience Research. 1992;32:51-59
Biglari A, Bataille D, Naumann U, Weller M, Zirger J, Castro MG, Lowenstein PR. Effects of ectopic decorin in modulating intracranial glioma progression in vivo, in a rat syngeneic model. Cancer Gene Ther. 2004;11:721-732
Grant DS, Yenisey C, Rose RW, Tootell M, Santra M, Iozzo RV. Decorin suppresses tumor cell-mediated angiogenesis. Oncogene. 2002;21:4765-4777Davies et al 2004

## Claims

1. A transmembrane, transmucosal or transepithelial delivery system for use in a method of treating an eye disease, wherein the system comprises:
a pharmaceutically active agent which is a negatively charged polypeptide or glycoprotein; and
a polyarginine of 4 to 20 amino acids in length;
wherein the pharmaceutically active agent is mixed with or non-covalently bound to the polyarginine.

2. The system for use according to claim 1, wherein the polyarginine consists of 6, 7 or 10 arginine residues.

3. The system for use according to claim 1 or 2, wherein the pharmaceutically active agent has a molecular weight of up to 500 kDa.

4. The system for use according to any one of claims 1 to 3, wherein the pharmaceutically active agent is an anti-inflammatory compound, an anti-scarring drug or an antimicrobial compound.

5. The system for use according to any one of claims 1 to 4, which further comprises or is used in combination with one or more penetration enhancers, such as polyethylene glycol, fatty acid esters, diacids and/or monomethyl esters.

6. The system for use according to any one of claims 1 to 5, which is applied to the eye via an eye drop.

7. The system for use according to any one of claims 1 to 6, which transports the pharmaceutically active agent across the surface of the eye.

8. The system for use according to any one of claims 1 to 7, wherein the eye disease is age-related macular degeneration scarring, proliferative vitreoretinopathy or glaucoma.

## Patentansprüche

1. Transmembran-, transmucosales oder transepitheliales Liefersystem zur Verwendung in einem Verfahren zur Behandlung einer Augenkrankheit, wobei das System umfasst:
ein pharmazeutisch aktives Agens, das ein negativ geladenes Polypeptid oder Glykoprotein ist; und
ein Polyarginin von 4 bis 20 Aminosäuren Länge;
wobei das pharmazeutisch aktive Agens mit dem Polyarginin gemischt oder nicht-kovalent an das Polyarginin gebunden ist.

2. System zur Verwendung gemäß Anspruch 1, wobei das Polyarginin aus 6, 7 oder 10 Argininresten besteht.

3. System zur Verwendung gemäß Anspruch 1 oder 2, wobei das pharmazeutisch aktive Agens ein Molekulargewicht von bis zu 500 kDa hat.

4. System zur Verwendung gemäß einem beliebigen der Ansprüche von 1 bis 3, wobei das pharmazeutisch aktive Agens eine entzündungshemmende Verbindung, ein Anti-Vernarbungsarzneimittel oder eine antimikrobielle Verbindung ist.

5. System zur Verwendung gemäß einem beliebigen der Ansprüche von 1 bis 4, das ferner einen oder mehrere Penetrationsverstärker, wie Polyethylenglykol, Fettsäureester, Disäuren und/oder Monomethylester umfasst oder in Kombination mit diesen verwendet wird.

6. System zur Verwendung gemäß einem beliebigen der Ansprüche von 1 bis 5, das an das Auge über einen Augentropfen verabreicht wird.

7. System zur Verwendung gemäß einem beliebigen der Ansprüche von 1 bis 6, das das pharmazeutisch aktive Agens über die Augenoberfläche transportiert.

8. System zur Verwendung gemäß einem beliebigen der Ansprüche von 1 bis 7, wobei die Augenkrankheit eine altersbedingte Makuladegenerations-Vernarbung, proliferative Vitreoretinopathie oder Glaukom ist.

## Revendications

1. Système d'administration transmembranaire, transmuqueuse ou transépithéliale pour son utilisation dans un procédé de traitement d'une maladie oculaire, où le système comprend :
un agent pharmaceutiquement actif qui est un polypeptide négativement chargé ou une glycoprotéine ; et
une polyarginine d'une longueur de 4 à 20 acides aminés ;
dans lequel l'agent pharmaceutiquement actif est mélangé avec ou lié de façon non covalente à la polyarginine.

2. Système pour son utilisation selon la revendication 1, dans lequel la polyarginine consiste en 6, 7 ou 10 résidus arginine.

3. Système pour son utilisation selon la revendication 1 ou 2, dans lequel l'agent pharmaceutiquement actif possède un poids moléculaire allant jusqu'à 500 kDa.

4. Système pour son utilisation selon l'une quelconque des revendications 1 à 3, dans lequel l'agent pharmaceutiquement actif est un composé anti-inflammatoire, un médicament anti-cicatrices ou un composé antimicrobien.

5. Système pour son utilisation selon l'une quelconque des revendications 1 à 4, qui comprend en outre ou est utilisé en combinaison avec un ou plusieurs agents améliorant la pénétration, tel que le polyéthylène glycol, des esters d'acide gras, des diacides et/ou des esters monométhyliques.

6. Système pour son utilisation selon l'une quelconque des revendications 1 à 5, qui est appliqué à l'œil via une goutte oculaire.

7. Système pour son utilisation selon l'une quelconque des revendications 1 à 6, qui transporte l'agent pharmaceutiquement actif à travers la surface de l'œil.

8. Système pour son utilisation selon l'une quelconque des revendications 1 à 7, où la maladie oculaire est une cicatrice provoquée par la dégénérescence maculaire liée à l'âge, la vitréorétinopathie proliférante ou le glaucome.
